# EUROPEAN PATENT APPLICATION

(11) **EP 4 209 233 A1**
(43) Date of publication of application: **12.07.2023**
(21) Application number: 22174221.6
(22) Date of filing: 19.05.2022
(51) Int. Cl.: A61M 1/00, A61B 18/14, F16K 3/24, F16K 3/32

(54) **FLOW CONTROLLABLE TYPE SUCTION AND IRRIGATION DEVICE**

(30) Priority: 06.01.2022 KR 20220002375
(71) Applicant: Orange Medics, Inc., useong-gu Daejeon 34138 (KR)
(72) Inventor: LEE, Ki Seok, 34190 Daejeon (KR)
(74) Representative: Vossius & Partner Patentanwälte Rechtsanwälte mbB

(57) **Abstract**

Disclosed is a flow controllable type suction and irrigation device that allows easy manipulation and flow control while improving convenience of use. The flow controllable type suction and irrigation device includes a handle, a cannula, and a suction supply unit. The handle has an installation space therein. The cannula includes a conductive tube extending in a forward direction of the handle to be inserted into the abdominal cavity of a patient and coupled to an electrode for surgery, and an insulating protective tube disposed to surround the conductive tube. The suction supply unit is provided to the handle and supplies an irrigation fluid to the cannula or suctions blood or contaminants from the abdominal cavity of the patient through the cannula. The suction supply unit includes a first chamber having a first through-hole, a second chamber connected to the first chamber through a communication hole and having a second through-hole, a first rotary valve disposed inside the first chamber and regulating an open area of the first through-hole while rotating, and a second rotary valve disposed inside the second chamber and regulating an open area of the second through-hole while rotating.

## Description

### FIELD

The present invention relates to a flow controllable type suction and irrigation device, and, more particularly, to a flow controllable type suction and irrigation device that allows easy manipulation and flow control while improving convenience of use.

### BACKGROUND

In general, endoscopic (or laparoscopic) surgery is a minimally invasive surgery and has been rapidly developed in recent years due to advantages, such as reduction of pain, infection, wounds, hospitalization period, and the like.

In laparoscopic surgery, a surgical instrument, that is, a laparoscopic suction and irrigation system, is used to supply the body of the patient with an irrigation fluid to clean a surgical site of a patient, and to discharge contaminants from the body of the patient after washing the surgical site.

A typical laparoscopic suction and irrigation system includes a cannula inserted into a patient body, a system body having a mixing chamber connected to the cannula, a cleaning flow channel connected to the mixing chamber to supply an irrigation fluid into the patient body, and a discharge flow channel adapted to discharge contaminants from the patient body.

In recent years, the laparoscopic suction and irrigation system further includes a component that enables electric surgery for incision of a surgical site and the like.

Typically, in laparoscopic surgery using the laparoscopic suction and irrigation system, electric surgery, supply of an irrigation fluid and discharge of contaminants are rapidly carried out. In recent years, development of laparoscopic surgery technology enables complicated and microscopic surgery, such as cancer surgery and the like.

However, the typical suction and irrigation system can generate excessive suction force, which blocks the cannula due to adhesion of surgical tissue thereto, causing obstruction to surgery or damage to the tissue adhered to the cannula. In addition, the typical suction and irrigation system often provides inconvenience in surgery due to excessive supply of the irrigation fluid, causing delay of surgery.

Therefore, there is a need for a laparoscopic suction and irrigation system that enables regulation of suction force in order to improve surgery efficiency, as needed, while allowing easy control of a flow rate of an irrigation fluid through regulation of cleaning pressure.

One example of the related art is disclosed in Korean Patent Registration No. 1837055 (Issue Date: 2018.03.09.)

### SUMMARY

Embodiments of the present invention are conceived to solve such problems in the art and it is an aspect of the present invention to provide a flow controllable type suction and irrigation device that allows easy manipulation and flow control while improving convenience of use.

It will be understood that aspects of the present invention are not limited to the above and the above and other aspects of the present invention will become apparent to those skilled in the art from the detailed description of the following embodiments in conjunction with the accompanying drawings.

In accordance with one aspect of the present invention, there is provided a flow controllable type suction and irrigation device including: a handle having an installation space therein; a cannula provided with a conductive tube extending in a forward direction of the handle to be inserted into the abdominal cavity of a patient and coupled to an electrode for surgery and an insulating protective tube disposed to surround the conductive tube; and a suction supply unit provided to the handle and supplying an irrigation fluid to the cannula or suctioning blood or contaminants from the abdominal cavity of the patient through the cannula, wherein the suction supply unit includes a first chamber having a first through-hole, a second chamber connected to the first chamber through a communication hole and having a second through-hole, a first rotary valve disposed inside the first chamber and regulating an open area of the first through-hole while rotating, and a second rotary valve disposed inside the second chamber and regulating an open area of the second through-hole while rotating.

The first rotary valve may include: a first body spaced apart from an inner peripheral surface of the first chamber; a first sealing ring surrounding a lower region of the first body; a second sealing ring surrounding an upper region of the first body; and a first ring valve disposed on the first sealing ring to protrude in a circumferential direction of the first body, the first ring valve being formed to closely contact the inner peripheral surface of the first chamber and regulating the open area of the first through-hole while rotating together with the first body.

The first rotary valve may further include a first stationary flange protruding from a lower end of the first body in the circumferential direction of the first body, and a pair of second stationary flanges spaced apart from each other and protruding from the upper region of the first body in the circumferential direction of the first body.

The first sealing ring may be disposed between the first stationary flange and the first ring valve, and the second sealing ring may be disposed between the pair of second stationary flanges.

The first ring valve may have a lower surface formed in the circumferential direction beneath a lower end of the first through-hole and an upper surface slanted upwards in a first rotation direction such that a height of the first ring valve gradually increases in the first rotation direction.

The open area of the first through-hole opened by the first ring valve may be increased as the first rotary valve is rotated in the first rotation direction, and may be decreased as the first rotary valve is rotated in an opposite direction to the first rotation direction.

The suction supply unit may include: a first connection port connected to the first through-hole and extending in a backward direction of the first chamber to be connected to a supply tube; a second connection port connected to the second through-hole and extending in a backward direction of the second chamber to be connected to a suction tube; and a third connection port connected to the communication hole and extending in a forward direction of the first chamber and the second chamber to be connected to the cannula.

The flow controllable type suction and irrigation device may further include a switch unit provided to the handle and supplying electricity to the conductive tube.

The switch unit may include: a stationary base coupled to the handle in the installation space and closely contacting the third connection port; a switch provided to the stationary base, connected to an electric line through which electricity is supplied, and exposed outside the handle; and an electrode clip electrically connected to the switch in the installation space and inserted into a slit formed through the third connection port to contact the conductive tube.

The stationary base may include a support flange formed corresponding to an outer peripheral surface of the third connection port and formed with a rounded section closely contacting the third connection port.

The handle may include a first handle case and a second handle case, and the switch unit may be disposed biased to one side of the second handle case.

The second handle case may include: an opening formed through the second handle case to expose the switch; an upper shaft cover receiving an upper portion of a rotational shaft provided to the switch, wherein the rotational shaft constitutes a center of rotation of the switch; and a guide wall formed along a circumference of the opening inside the second handle case.

The stationary base may include a coupling plate formed to closely contact the guide wall, and a lower shaft cover formed on the coupling plate to receive a lower portion of the rotational shaft, and coupled to the upper shaft cover.

The flow controllable type suction and irrigation device may further include: an adapter rotatably provided to the third connection port and coupled to one end of the conductive tube therein; a first knob disposed at a front side of the handle to reciprocate in an axial direction of the conductive tube and receiving a portion of the adapter in an interior space of the first knob; a second knob coupled to a front end of the first knob and coupled to a rear end of the protective tube therein, the second knob being integrally operated with the first knob; and a manipulation unit coupled to a rear end of the second knob and having an insert securing the rear end of the protective tube together with the second knob.

The protective tube comprises a tube expansion portion formed at the rear end portion of the protective tube. The tube expansion portion may be pressed and secured by the insert and a first pressing portion formed at a rear side of the second knob.

The suction supply unit may further include: a first turning head disposed at an upper end of the first rotary valve and transferring rotational force supplied from outside to the first rotary valve; and a second turning head disposed at an upper end of the second rotary valve and transferring rotational force supplied from outside to the second rotary valve.

According to embodiments of the present invention, the switch unit is disposed biased to one side of a central line of the handle, whereby a user can manipulate the suction supply unit with his or her thumb while holding the handle and operating the switch with his or her index finger, thereby improving manipulation convenience.

According to embodiments of the present invention, the first rotary valve and the second rotary valve allow accurate control of a flow rate of an irrigation fluid or suction force for suctioning blood or contaminants from the abdominal cavity of a patient through accurate control of rotation.

It will be understood that advantageous effects of the present invention are not limited to the above and include any advantageous effects conceivable from the features disclosed in the detailed description of the present invention or the appended claims.

### DRAWINGS

The above and other aspects, features, and advantages of the present invention will become apparent from the detailed description of the following embodiments in conjunction with the accompanying drawings:
FIG. 1 is a perspective view of a flow controllable type suction and irrigation device according to one embodiment of the present invention;
FIG. 2 is a front view of the flow controllable type suction and irrigation device shown in FIG. 1, with a portion of a handle case omitted;
FIG. 3 is a perspective view of a suction supply unit of the flow controllable type suction and irrigation device according to the embodiment of the present invention;
FIG. 4 is an exploded perspective view of the suction supply unit of the flow controllable type suction and irrigation device according to the embodiment of the present invention;
FIG. 5 is a perspective sectional view of a first chamber and a second chamber of the flow controllable type suction and irrigation device according to the embodiment of the present invention;
FIG. 6 is a perspective sectional view of the first chamber, the second chamber, and a third connection port of the flow controllable type suction and irrigation device according to the embodiment of the present invention;
FIG. 7 is a plan view of the suction supply unit of the flow controllable type suction and irrigation device according to the embodiment of the present invention;
FIG. 8 is an exemplary view illustrating an open area of a first through-hole according to a rotated state of a first rotary valve of the flow controllable type suction and irrigation device according to the embodiment of the present invention;
FIG. 9 is a cross-sectional view of the first chamber and the first rotary valve of the flow controllable type suction and irrigation device according to the embodiment of the present invention;
FIG. 10 is a perspective view of a manipulation unit of the flow controllable type suction and irrigation device according to the embodiment of the present invention;
FIG. 11 is an exploded perspective view of the manipulation unit shown in FIG. 10;
FIG. 12 is an exploded perspective view of the manipulation unit shown in a different direction from FIG. 11;
FIG. 13 is an exemplary sectional view illustrating operation of the manipulation unit of the flow controllable type suction and irrigation device according to the embodiment of the present invention;
FIG. 14 is an exploded perspective view of a switch unit of the flow controllable type suction and irrigation device according to the embodiment of the present invention;
FIG. 15 is an exemplary view of the switch unit of the flow controllable type suction and irrigation device according to the embodiment of the present invention; and
FIG. 16 is a perspective sectional view of an electrode clip and a conductive tube of the flow controllable type suction and irrigation device according to the embodiment of the present invention.

### DETAILED DESCRIPTION

Hereinafter, exemplary embodiments of the present invention will be described in detail with reference to the accompanying drawings. It should be understood that the present invention is not limited to the following embodiments and may be embodied in different ways, and that the embodiments are provided for complete disclosure and thorough understanding of the present invention by those skilled in the art. In addition, description of unrelated components is omitted for clear description of the present invention and like components will be denoted by like reference numerals throughout the specification.

When an element is referred to as being "connected to, or "coupled to" another element, it may be directly or indirectly connected or coupled to the other element or intervening elements may be present. However, when an element or layer is referred to as being "directly on," "directly connected to," or "directly coupled to" another element or layer, there are no intervening elements or layers present.

The terminology used herein is for the purpose of describing particular embodiments and is not intended to be limiting. As used herein, the singular forms, "a," "an," and "the" are intended to include the plural forms as well, unless the context clearly indicates otherwise. Moreover, the terms "comprises," "comprising," "includes," and/or "including," when used in this specification, specify the presence of stated features, integers, steps, operations, elements, components, and/or groups thereof, but do not preclude the presence or addition of one or more other features, integers, steps, operations, elements, components, and/or groups thereof.

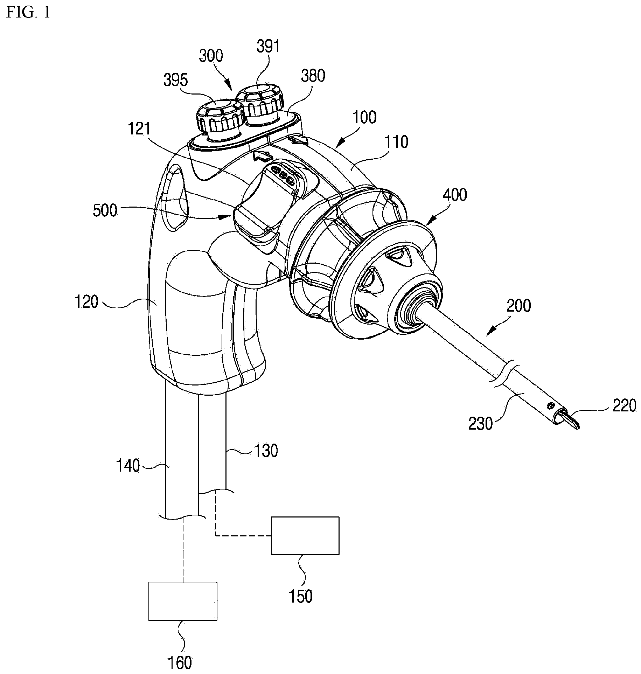

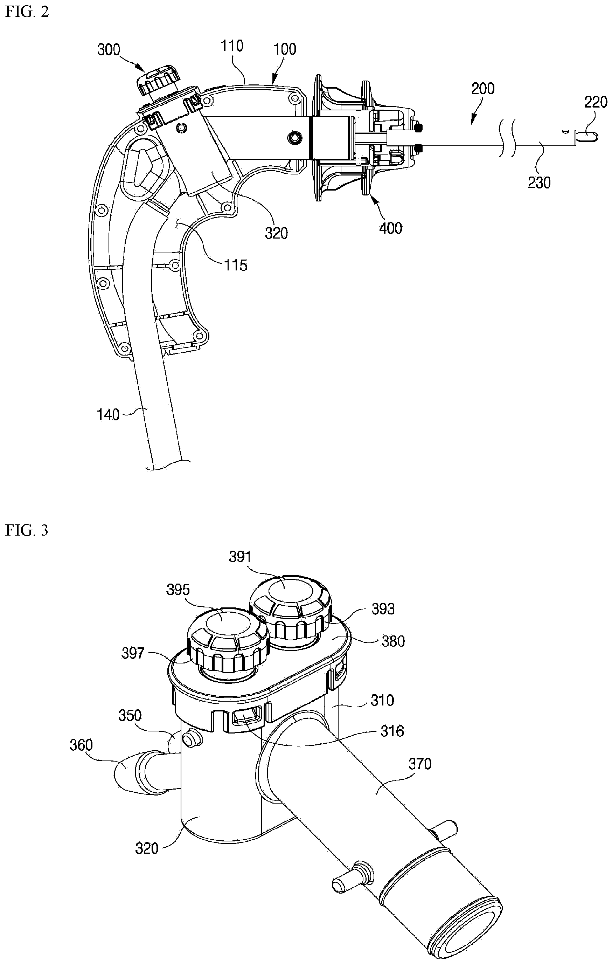

FIG. 1 is a perspective view of a flow controllable type suction and irrigation device according to one embodiment of the present invention and FIG. 2 is a front view of the flow controllable type suction and irrigation device shown in FIG. 1, with a portion of a handle case omitted.

Referring to FIG. 1 and FIG. 2, the flow controllable type suction and irrigation device according to the embodiment of the present invention may include a handle 100, a cannula 200, and a suction supply unit 300.

The handle 100 may include a pair of handle cases. The pair of handle cases may include a first handle case 110 and a second handle case 120, which may have an asymmetrical shape. By way of example, unlike the first handle case 110, the second handle case 120 may be formed with an opening 121. Except for the opening 121 of the second handle case 120, the first handle case 110 and the second handle case 120 may be bilaterally symmetrical as a whole. The handle 100 may be gripped by a user hand and may have an installation space 115 therein.

Hereinafter, for convenience of description, a front end/front portion/forward direction will be referred to with reference to a direction from the handle 100 towards the cannula 200 and a rear end/rear portion/backward direction will be referred to with reference to an opposite direction to the direction from the handle 100 towards the cannula 200.

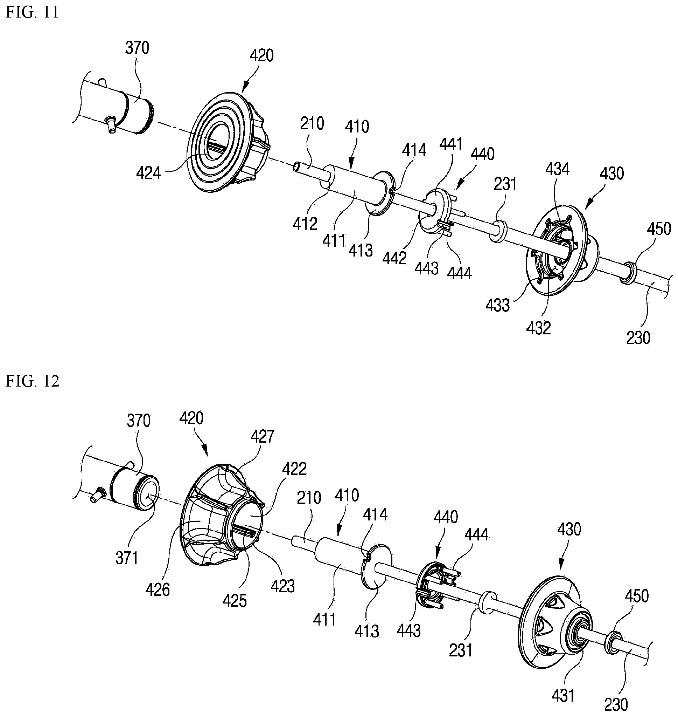

The cannula 200 extends in the forward direction of the handle 100 and may be inserted into the abdominal cavity of a patient. The cannula 200 may include a conductive tube 210 (see FIG. 11), to which an electrode 220 for surgery is coupled, and an insulating protective tube 230 surrounding the conductive tube.

The suction supply unit 300 is disposed in the installation space 115 and serves to supply an irrigation fluid to the cannula 200 or to suction blood or contaminants from the abdominal cavity of a patient through the cannula 200.

The suction supply unit 300 may be connected to an external power supply 150 through a supply tube 130. For example, the external power supply 150 may be a pump. The irrigation fluid may be supplied by the external power supply 150 and may flow to the conductive tube of the cannula 200 through the suction supply unit 300 to be supplied to the abdominal cavity of the patient.

The suction supply unit 300 may be connected to an external suction unit 160 through a suction tube 140. When suction force is generated by the external suction unit 160, blood or contaminants may be suctioned from the abdominal cavity of the patient to the conductive tube of the cannula 200 and then discharged after flowing to the suction tube 140 through the suction supply unit 300.

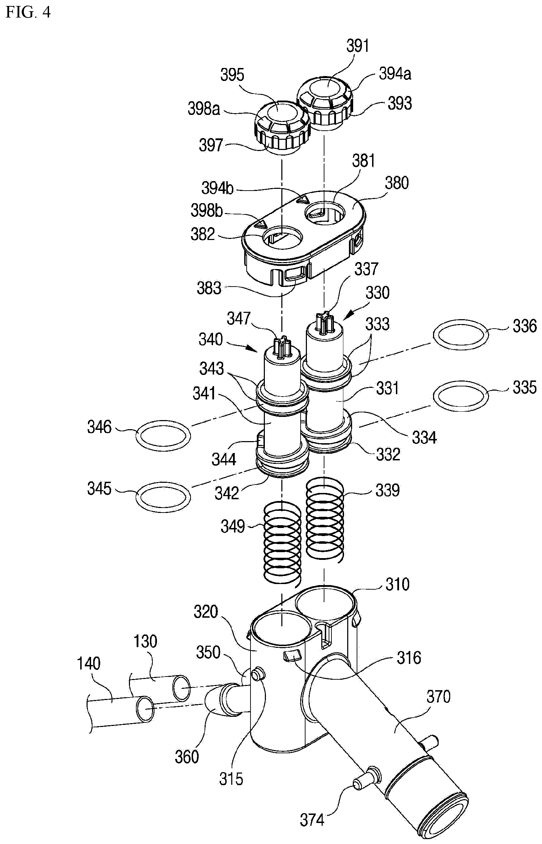

In particular, a first rotary valve 330 (see FIG. 4) and a second rotary valve 340 (see FIG. 4) are rotatable and allow accurate regulation of rotation, thereby allowing accurate control of a flow rate of the irrigation fluid or suction force for suctioning blood or contaminants from the abdominal cavity of the patient.

In addition, the flow controllable type suction and irrigation device may include a manipulation unit 400 and a switch unit 500.

The manipulation unit 400 may be disposed at a front side of the handle 100 and may allow the protective tube 230 to expose or cover the electrode 220 while reciprocating in an axial direction of the conductive tube. In addition, the manipulation unit 400 may rotate the electrode 220 by allowing rotation of the conductive tube while rotating.

The switch unit 500 may have one end disposed in the installation space 115 and the other end exposed through the opening 121 formed on the second handle case 120. The switch unit 500 may block supply of electric power to the conductive tube or allow supply of different magnitudes of electric power thereto. As a result, the electrode 220 can perform electric surgery, such as incision, coagulation and the like of a patient's surgery site.

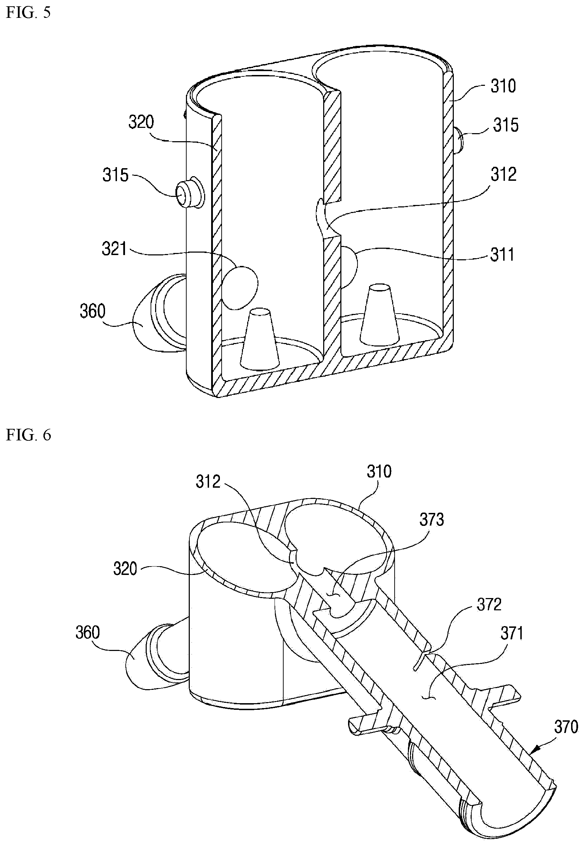

FIG. 3 is a perspective view of a suction supply unit of the flow controllable type suction and irrigation device according to the embodiment of the present invention; FIG. 4 is an exploded perspective view of the suction supply unit of the flow controllable type suction and irrigation device according to the embodiment of the present invention; FIG. 5 is a perspective sectional view of a first chamber and a second chamber of the flow controllable type suction and irrigation device according to the embodiment of the present invention; and FIG. 6 is a perspective sectional view of the first chamber, the second chamber, and a third connection port of the flow controllable type suction and irrigation device according to the embodiment of the present invention.

Referring to FIG. 3 to FIG. 6, the suction supply unit 300 may include a first chamber 310, a second chamber 320, a first rotary valve 330, a second rotary valve 340, a first connection port 350, a second connection port 360, and a third connection port 370.

Each of the first chamber 310 and the second chamber 320 may have a barrel shape open at an upper side thereof. The first chamber 310 and the second chamber 320 may be disposed to be adjacent each other and the interior of the first chamber 310 may communicate with the interior of the second chamber 320 through a communication hole 312.

The first chamber 310 may have a first through-hole 311. The first connection port 350 may be connected to the first through-hole 311 and may extend from a rear side of the first chamber 310 to be connected to the supply tube 130.

The second chamber 320 may have a second through-hole 321. The second connection port 360 may be connected to the second through-hole 321 and may extend from a rear side of the second chamber 320 to be connected to the suction tube 140.

Each of the first chamber 310 and the second chamber 320 may be formed on an outer peripheral surface thereof with a first coupling protrusion 315 and a second coupling protrusion 316.

At least part of the first rotary valve 330 may be disposed in an interior space of the first chamber 310.

The first rotary valve 330 may include a first body 331, a first sealing ring 335, a second sealing ring 336, and a first ring valve 334. In addition, the first rotary valve 330 may further include a first stationary flange 332, second stationary flanges 333, and a first resilient member 339.

The first body 331 may form a body of the first rotary valve 330. An outer diameter of the first body 331 may be smaller than an outer diameter of the first chamber 310. Accordingly, when the first body 331 is inserted into the first chamber 310, an outer peripheral surface of the first body 331 may be spaced apart from an inner peripheral surface of the first chamber 310.

The first sealing ring 335 may be disposed to surround a lower region of the first body 331 to seal a space between an outer peripheral surface of the lower region of the first body 331 and an inner peripheral surface of a lower region of the first chamber 310.

The second sealing ring 336 may be disposed to surround an upper region of the first body 331 to seal a space between an outer peripheral surface of the upper region of the first body 331 and an inner peripheral surface of an upper region of the first chamber 310.

The first ring valve 334 may be disposed on the first sealing ring 335 and may protrude from the first body 331 in a circumferential direction of the first body 331. The first ring valve 334 is formed to closely contact an inner peripheral surface of the first chamber 310 and may regulate an open area of the first through-hole 311 while rotating together with the first body 331.

A protruding thickness of the first ring valve 334 may be substantially the same as a radial distance between an outer peripheral surface of the first body 331 and the inner peripheral surface of the first chamber 310 such that the first ring valve 334 closely contacts the inner peripheral surface of the first chamber 310.

The first stationary flange 332 may protrude from a lower end of the first body 331 in the circumferential direction thereof.

The second stationary flanges 333 may be provided as a pair and may be spaced apart from each other and protrude from the upper region of the first body 331 in the circumferential direction thereof.

The first sealing ring 335 may be disposed between the first stationary flange 332 and the first ring valve 334 to closely contact the inner peripheral surface of the first chamber 310.

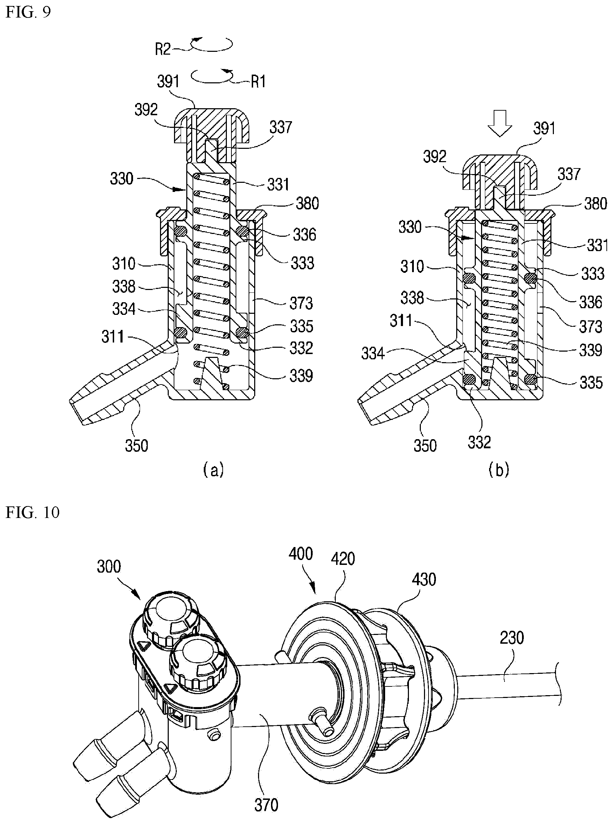

The second sealing ring 336 may be disposed between the pair of second stationary flanges 333 to closely contact the inner peripheral surface of the first chamber 310. Accordingly, a first flow channel space 338 (see FIG. 9) may be defined between the first body 331 and the first chamber 310. Specifically, the first flow channel space 338 may be a space surrounded by the first ring valve 334, a lower stationary flange of the second stationary flanges 333, the first body 331, and the first chamber 310.

At least part of the first resilient member 339 may be received in the interior space of the first body 331. A lower end of the first resilient member 339 may closely contact a bottom surface of the first chamber 310 and an upper end of the first resilient member 339 may closely contact an upper surface of the interior space of the first body 331 to resiliently support the first body 331 in an upward direction. In an initial state in which external force is not applied to the first body 331, the first body 331 may be in a state of being moved upwards by the first resilient member 339 (see FIG. 9).

The second rotary valve 340 may have the same shape as the first rotary valve 330.

That is, at least part of the second rotary valve 340 may be disposed in the interior space of the second chamber 320.

The second rotary valve 340 may include a second body 341, a third stationary flange 342, fourth stationary flanges 343, a second ring valve 344, a third sealing ring 345, a fourth sealing ring 346, and a second resilient member 349.

The second body 341 may form a body of the second rotary valve 340. An outer diameter of the second body 341 may be smaller than an outer diameter of the second chamber 320. Accordingly, when the second body 341 is inserted into the second chamber 320, an outer peripheral surface of the second body 341 may be spaced apart from an inner peripheral surface of the second chamber 320.

The third stationary flange 342 may protrude from a lower end of the second body 341 in a circumferential direction thereof.

The fourth stationary flanges 343 may be provided as a pair and may be spaced apart from each other and protrude from the upper region of the second body 341 in the circumferential direction thereof.

The second ring valve 344 may protrude from a lower region of the second body 341 in the circumferential direction thereof. The second ring valve 344 may be formed to closely contact an inner peripheral surface of the second chamber 320 and may be rotated together with the second body 341.

The third sealing ring 345 may be disposed between the third stationary flange 342 and the second ring valve 344 to closely contact the inner peripheral surface of the second chamber 320.

The fourth sealing ring 346 may be disposed between the pair of fourth stationary flanges 343 to closely contact the inner peripheral surface of the second chamber 320. Accordingly, a second flow channel space (not shown) may be defined between the second body 341 and the second chamber 320. Specifically, the second flow channel space may be a space surrounded by the second ring valve 344, a lower stationary flange of the fourth stationary flanges 343, the second body 341, and the second chamber 320. As described above, since the second rotary valve 340 may have the same shape as the first rotary valve 330, the second flow channel space may have the same shape as the first flow channel space 338.

At least part of the second resilient member 349 may be received in an interior space of the second body 341. A lower end of the second resilient member 349 may closely contact a bottom surface of the second chamber 320 and an upper end of the second resilient member 349 may closely contact an upper surface of the interior space of the second body 341 to resiliently support the second body 341 in an upward direction. In an initial state in which external force is not applied to the second body 341, the second body 341 may be in a state of being moved upwards by the second resilient member 349 (see FIG. 9).

The suction supply unit 300 may include a cap 380, a first turning head 391, and a second turning head 395.

The cap 380 may be coupled to upper ends of the first chamber 310 and the second chamber 320 to cover the first chamber 310 and the second chamber 320.

The cap 380 may have a first open hole 381, a second open hole 382, and coupling holes 383.

The first open hole 381 may be formed concentric with the first chamber 310. An inner diameter of the first open hole 381 may be greater than an outer diameter of the first body 331. Accordingly, the first body 331 may be moved upwards or downwards through the first open hole 381.

The second open hole 382 may be formed concentric with the second chamber 320. An inner diameter of the second open hole 382 may be greater than an outer diameter of the second body 341. Accordingly, the second body 341 may be moved upwards or downwards through the second open hole 382.

When the second coupling protrusions 316 of the first chamber 310 and the second chamber 320 are coupled to the coupling holes 383 of the cap 380, the cap 380 can be firmly coupled to the upper ends of the first chamber 310 and the second chamber 320.

The first body 331 may be formed at the upper end thereof with a first coupling member 337 and the second body 341 may be formed at the upper end thereof with a second coupling member 347. Each of the first coupling member 337 and the second coupling member 347 may have a "+"-shaped cross-section.

The first turning head 391 may have a first insertion groove 392 coupled to the first coupling member 337. The first turning head 391 may transfer rotational force from outside to the first rotary valve 330. That is, when a user turns the first turning head 391, the first rotary valve 330 may rotate in place without moving up and down. In addition, when a user presses the first turning head 391 in a downward direction, the first rotary valve 330 may also be moved downwards.

Further, the second turning head 395 may have a second insertion groove (not shown) coupled to the second coupling member 347. The second turning head 395 may transfer rotational force from outside to the second rotary valve 340. That is, when a user turns the second turning head 395, the second rotary valve 340 may rotate in place without moving up and down. In addition, when a user presses the second turning head 395 in the downward direction, the second rotary valve 340 may also be moved downwards. As a whole, the second turning head 395 may have the same shape as the first turning head 391.

The third connection port 370 may be connected to front surfaces of the first chamber 310 and the second chamber 320, and may extend forwards from the front surfaces of the first chamber 310 and the second chamber 320. The third connection port 370 may be provided therein with an accommodation flow channel 371, which may be connected to the communication hole 312 by a connection flow channel 373.

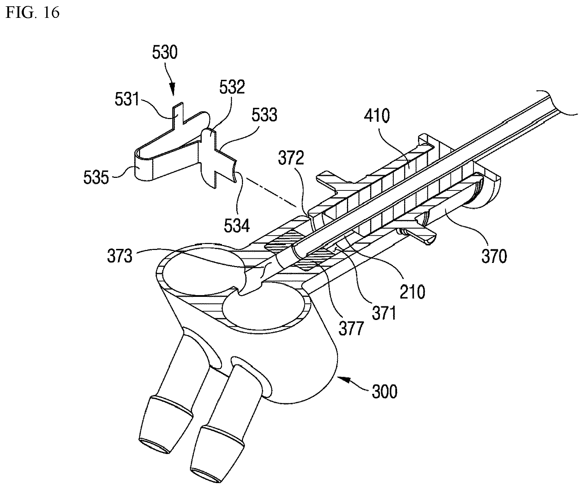

The third connection port 370 may have a slit 372. The slit 372 may be formed through the third connection port 370 to be connected to the accommodation flow channel 371. Referring to FIG. 16, the accommodation flow channel 371 may be provided with a channel sealing member 377. The channel sealing member 377 may be disposed to surround a proximal end of the conductive tube 210 and may connect the connection flow channel 373 to the conductive tube 210 to prevent a fluid from leaking to the accommodation flow channel 371 at a connection site between the connection flow channel 373 and the conductive tube 210.

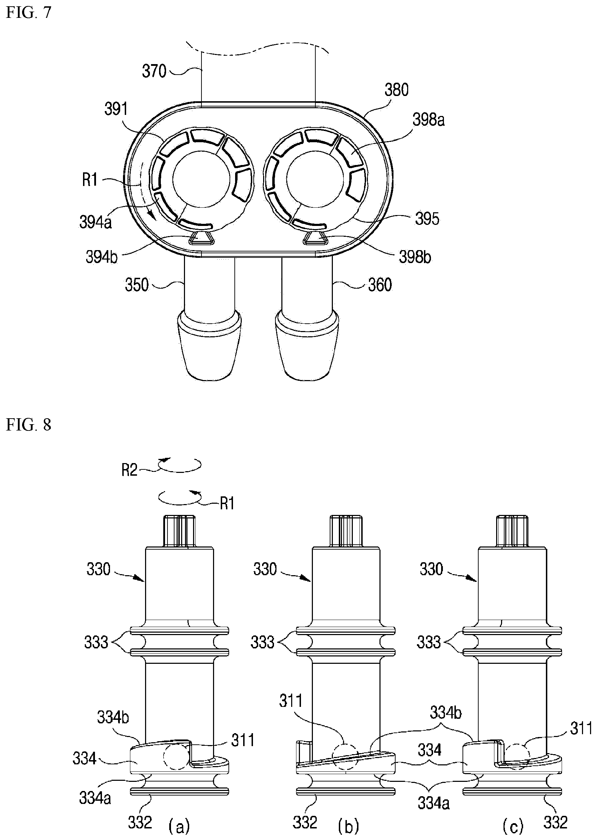

FIG. 7 is a plan view of the suction supply unit of the flow controllable type suction and irrigation device according to the embodiment of the present invention; FIG. 8 is an exemplary view illustrating an open area of a first through-hole according to a rotated state of a first rotary valve of the flow controllable type suction and irrigation device according to the embodiment of the present invention; and FIG. 9 is a cross-sectional view of the first chamber and the first rotary valve of the flow controllable type suction and irrigation device according to the embodiment of the present invention. The flow controllable type suction and irrigation device according to the embodiment will be further described with reference to FIG. 7 to FIG. 9.

A lower surface 334a of the first ring valve 334 may be formed beneath a lower end of the first through-hole 311 in the circumferential direction. In addition, an upper surface 334b of the first ring valve 334 may be formed to have an upward slope in a first rotation direction R1. Accordingly, the first ring valve 334 may have a height gradually increasing in the first rotation direction R1.

FIG. 8(a) shows the first ring valve 334 completely blocking the first through-hole 311. From this state, when the first rotary valve 330 is rotated in the first rotation direction R1 as shown in FIG. 8(b), an open area of the first through-hole 311 opened by the first ring valve 334 is increased. As a result, the flow rate of the irrigation fluid flowing into the first chamber 310 through the first through-hole 311 is increased. Then, when the first rotary valve 330 is completely rotated in the first rotation direction R1, as shown in FIG. 8(c), the open area of the first through-hole 311 can be maximized to provide a maximum flow rate of the irrigation fluid flowing therethrough. As the open area of the first through-hole 311 is increased by the first ring valve 334, a large amount of the irrigation fluid flows to the conductive tube through the third connection port 370 to be supplied to the abdominal cavity of a patient.

On the contrary, as the first rotary valve 330 is rotated from the state shown in FIG. 8(c) in a second rotation direction R2 opposite to the first rotation direction R1, the open area of the first through-hole 311 opened by the first ring valve 334 is decreased. Then, when the first rotary valve 330 is completely rotated in the second rotation direction R2, the first through-hole 311 is completely closed by the first ring valve 334 to stop flow of the irrigation fluid. A user can easily regulate a supply amount of the irrigation fluid through control of the degree of rotation of the first rotary valve 330.

The first rotary valve 330 and the second rotary valve 340 may be operated in the same manner.

That is, the second rotary valve 340 may also regulate the open area of the second through-hole 321 in the same way as the method of regulating the open area of the first through-hole 311 by the first rotary valve 330. Since suction force increases with increasing open area of the second through-hole 321 opened by the second ring valve 344, a relatively large amount of blood or contaminants may be suctioned from the abdominal cavity of the patient into the third connection port 370 through the conductive tube and may flow through the second chamber 320, the second through-hole 321, the second connection port 360 and the suction tube 140.

On the contrary, since suction force decreases with decreasing open area of the second through-hole 321 opened by the second ring valve 344, a relatively small amount of blood or contaminants may be suctioned from the abdominal cavity of the patient. As such, a user can easily regulate suction of blood or contaminants from the abdominal cavity of the patient through control of the degree of rotation of the second rotary valve 340.

In an initial state in which the first rotary valve 330 is moved upwards by the first resilient member 339, when a user turns the first turning head 391 to regulate the degree of rotation of the first rotary valve 330 (see FIG. 9(a)) and then presses the first turning head 391 to move the first rotary valve 330 in the downward direction, the flow rate of the irrigation fluid flowing into the first chamber 310 through the first through-hole 311 can be regulated.

Then, when the user does not press the first turning head 391, the first rotary valve 330 is moved upwards to return to the initial state by the first resilient member 339. When the first rotary valve 330 returns to the initial state, the first through-hole 311 is opened whereas the first through-hole 311 is not connected to the connection flow channel 373. As a result, supply of the irrigation fluid is stopped.

Likewise, in an initial state in which the second rotary valve 340 is moved upwards by the second resilient member 349, when a user turns the second turning head 395 to regulate the degree of rotation of the second rotary valve 340 and then presses the second turning head 395 to move the second rotary valve 340 in the downward direction, the flow rate of the irrigation fluid flowing into the second chamber 320 through the second through-hole 321 can be regulated.

Then, when the user does not press the second turning head 395, the second rotary valve 340 is moved upwards to return to the initial state by the second resilient member 349. When the second rotary valve 340 returns to the initial state, the second through-hole 321 is opened whereas the second through-hole 321 is not connected to the connection flow channel 373. As a result, suction operation is stopped.

As shown in FIG. 4, the first turning head 391 may include a first knurling 393.

The first knurling 393 may be formed on an outer peripheral surface of the first turning head 391. The first knurling 393 may serve to prevent user's fingers from slipping when the user turns the first turning head 391.

In addition, the suction supply unit 300 may have a first bump display portion.

The first bump display portion may include a first display portion 394a formed on an upper surface of the first turning head 391 and a first reference portion 384b formed on an upper surface of the cap 380. The first display portion 394a may display increase or decrease of the flow rate of the irrigation fluid according to a rotating direction of the first rotary valve 330. By the first display portion 394a, it can be seen that the flow rate of the irrigation fluid is increased when the first turning head 391 is rotated in the first rotation direction R1.

The first turning head 391 can be rotated 360 degrees in the first rotation direction R1 in place. The suction supply unit 300 may be provided with a stopper (not shown) to prevent the first turning head 391 from rotating more than one turn in the same direction.

The second turning head 395 may have the same shape as the first turning head 391.

That is, the second turning head 395 may have a second knurling 397 on an outer peripheral surface thereof.

In addition, a second bump display portion may include a second display portion 398a formed on an upper surface of the second turning head 395 and a second reference portion 398b formed on an upper surface of the cap 380. The second display portion 398a may display increase or decrease of the flow rate of the irrigation fluid according to a rotating direction of the second rotary valve 340. By the second display portion 398a, it can be seen that suction force is increased when the second turning head 395 is rotated in the second rotation direction R1.

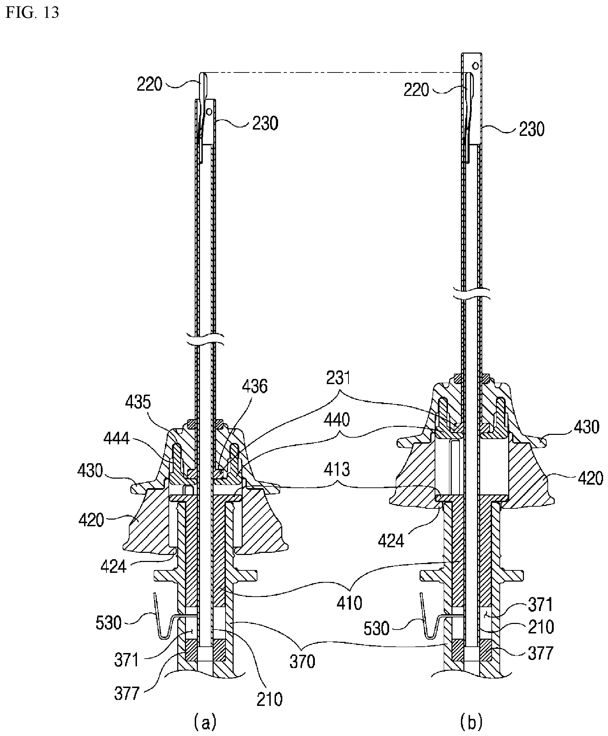

FIG. 10 is a perspective view of the manipulation unit of the flow controllable type suction and irrigation device according to the embodiment of the present invention; FIG. 11 is an exploded perspective view of the manipulation unit shown in FIG. 10; FIG. 12 is an exploded perspective view of the manipulation unit shown in a different direction from FIG. 11; and FIG. 13 is an exemplary sectional view illustrating operation of the manipulation unit of the flow controllable type suction and irrigation device according to the embodiment of the present invention.

Referring to FIG. 10 to FIG. 13, the manipulation unit 400 may include an adapter 410, a first knob 420, a second knob 430, and an insert 440.

The adapter 410 may include an adapter body 411 and a first latch flange 413.

The adapter body 411 may form a body of the adapter 410 and may have a first fitting hole 412 formed therethrough in an axial direction thereof. A rear end of the conductive tube 210 may be press-fitted into the first fitting hole 412, whereby the adapter 410 and the conductive tube 210 can be integrally rotated.

The adapter body 411 may be inserted into the accommodation flow channel 371 of the third connection port 370. The adapter 410 is secured to the third connection port 370 in an axial direction thereof. Although the adapter 410 is not moved in an axial direction of the third connection port 370, the adapter 410 can be rotated. The first latch flange 413 may be formed at a front end of the adapter body 411. The first latch flange 413 may be formed with a pair of grooves 414.

The first knob 420 is disposed at the front side of the handle and may receive a portion of the adapter 410 therein. Specifically, the first knob 420 may have a first accommodation hole 422, a front protrusion 423, a second latch flange 424, a first guide protrusion 425, grip recesses 426, and coupling ribs 427.

The first accommodation hole 422 may be formed through the first knob in the axial direction such that one end of the adapter 410 can be disposed in the accommodation flow channel 371 through the first accommodation hole 422. The first knob 420 may be reciprocated in the axial direction of the conductive tube 210.

The second latch flange 424 may be formed at a rear end of the first accommodation hole 422. When the second latch flange 424 is latched by the first latch flange 413 of the adapter 410 upon forward movement of the first knob 420, movement of the first knob 420 can be stopped.

The first guide protrusion 425 may be formed on an inner peripheral surface of the first accommodation hole 422. The first guide protrusion 425 may extend in the axial direction of the first knob and may be coupled to a first groove 414 of the adapter 410. Accordingly, the first knob 420 may be guided by the first guide protrusion 425 in the axial direction.

The coupling ribs 427 may be formed at constant intervals on the outer peripheral surface of the first knob 420 in the circumferential direction. In addition, each of the grip recesses 426 may be formed between the coupling ribs 427. The grip recesses 426 may be formed to allow a user to easily grip the first knob 420 with his or her fingers.

The front protrusion 423 may be formed at a front end of the first knob 420.

The second knob 430 may be coupled to the front end of the first knob 420.

The second knob 430 may include a second fitting hole 431, a second accommodation hole 432, a first coupling groove 433, a second guide protrusion 434, and a second coupling groove 435.

The second accommodation hole 432 may be formed through the second knob in an axial direction thereof and the front protrusion 423 of the first knob 420 may be inserted into a rear end of the second accommodation hole 432. Here, the coupling rib of the first knob 420 may be inserted into the first coupling groove 433. As a result, the first knob 420 and the second knob 430 may be linearly moved and rotated as an integral structure.

The second fitting hole 431 may be formed through the second knob inside the second accommodation hole 432 in the axial direction. The conductive tube 210 and the protective tube 230 may pass through the second fitting hole 431.

The second coupling groove 435 may be formed in the second accommodation hole 432 in the axial direction. The second coupling groove 435 may be provided in plural.

The insert 440 may be coupled to a rear end of the second knob 430.

The insert 440 may include an insert body 441, a through-hole 442, a second groove 443, and a third guide protrusion 444.

The insert body 441 may have an outer diameter corresponding to an inner diameter of the second accommodation hole 432 such that the insert body 441 can be inserted into the second accommodation hole 432.

The second groove 443 may be formed corresponding to the second guide protrusion 434 of the second knob 430. In addition, the third guide protrusion 444 may be formed corresponding to the second coupling groove 435 of the second knob 430. Accordingly, when the insert body 441 is inserted into the second accommodation hole 432, the second groove 443 may be coupled to the second guide protrusion 434 and the third guide protrusion 444 may be coupled to the second coupling groove 435. As a result, the insert 440 may be firmly secured to the second knob 430 and may be integrally moved and rotated together with the second knob 430.

On the other hand, the protective tube 230 includes a tube expansion portion 231 formed at the rear end portion of the protective tube 230.

The protective tube 230 may be inserted into the second fitting hole 431 at the rear side of the second knob 430 and the insert 440 may be coupled to the second accommodation hole 432 at the rear side of the protective tube 230.

The second accommodation hole 432 may be radially provided with a flat pressing portion 436 at a rear end of the second fitting hole 431.

When the insert 440 is coupled to the second accommodation hole 432 while pushing the tube expansion portion 231 in the forward direction, a front surface of the tube expansion portion 231 may be brought into close contact with the pressing portion 436 and a rear surface of the tube expansion portion 231 may be pressed by a front surface of the insert body 441, whereby the protective tube 230 can be reliably coupled to the second knob 430.

When the first knob 420 is moved forwards, the second knob 430 and the insert 440 may also be moved forwards together with the protective tube 230. As a result, the protective tube 230 may cover the electrode 220. In this state, when the first knob 420 is moved backwards, the second knob 430 and the insert 440 may also be moved backwards together with the protective tube 230. As a result, the electrode 220 may be exposed (see FIG. 13). In addition, when the first knob 420 is rotated, the adapter 410 and the conductive tube 210 . may be rotated. Also, when the conductive tube 210 is rotated, the electrode 220 may be rotated.

A securing ring 450 may be coupled to a front end of the second knob 430. The securing ring 450 can prevent blood or contaminants in the abdominal cavity of a patient from flowing into the second knob 430.

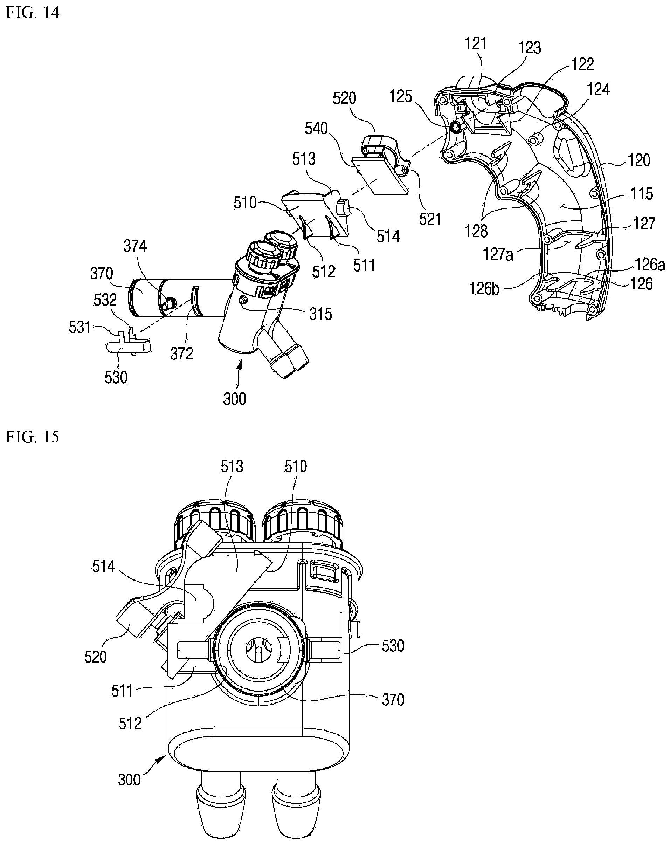

FIG. 14 is an exploded perspective view of a switch unit of the flow controllable type suction and irrigation device according to the embodiment of the present invention; FIG. 15 is an exemplary view of the switch unit of the flow controllable type suction and irrigation device according to the embodiment of the present invention; and FIG. 16 is a perspective sectional view of an electrode clip and the conductive tube of the flow controllable type suction and irrigation device according to the embodiment of the present invention.

As shown in FIG. 1, FIG. 2, and FIG. 14 to FIG. 16, the switch unit 500 may be provided to the handle 100 to supply electricity to the conductive tube 210.

The switch unit 500 may include a stationary base 510, a switch 520, and an electrode clip 530.

The stationary base 510 may closely contact the third connection port 370 in the installation space 115.

The switch 520 may be provided to the stationary base 510 and may be connected to an electric line (not shown) for supply of electricity from outside. A rotational shaft 521 may act as a rotational center of the switch 520. The switch 520 may be coupled to a substrate 540 and the stationary base 510 may support the substrate 540.

The stationary base 510 may have a support flange 511. The support flange 511 may be formed at a lower side of the stationary base 510. The support flange 511 may be formed with a rounded section 512 corresponding to an outer peripheral surface of the third connection port 370. As the stationary base 510 closely contacts an upper portion of the third connection port 370 and the rounded section 512 closely contacts the outer peripheral surface of the third connection port 370, the stationary base 510 can be stably secured thereto.

The stationary base 510 may have a coupling plate 513 and a lower shaft cover 514.

The coupling plate 513 may be formed at an upper portion of the stationary base 510 and the lower shaft cover 514 may be formed on the coupling plate 513. When the substrate 540 is secured to the stationary base 510, a lower portion of the rotational shaft 521 may be inserted into the lower shaft cover 514.

The second handle case 120 may have an opening 121, an upper shaft cover 123, and a guide wall 122.

The opening 121 may be formed through the second handle case 120 so as to correspond to the switch 520.

The upper shaft cover 123 and the guide wall 122 may protrude from an inner surface of the second handle case 120.

The upper shaft cover 123 may be formed near the opening 121. In addition, the guide wall 122 may be formed along the circumference of the opening 121.

When the switch 520 is coupled to the opening 121, the lower shaft cover 514 may be coupled to the upper shaft cover 123. Since a lower portion of the rotational shaft 521 is inserted into the lower shaft cover 514, the rotational shaft 521 may be inserted into the upper shaft cover 123 and the lower shaft cover 514.

In addition, when the switch 520 is coupled to the opening 121, the coupling plate 513 may be closely fitted into the guide wall 122. As a result, the stationary base 510 may be firmly secured between the second handle case 120 and the third connection port 370, and the switch 520 may be exposed from the second handle case 120 through the opening 121. Here, the switch unit 500 is disposed biased to one side of the second handle case 120.

As a result, the switch unit 500 is disposed biased to one side of a central line of the handle 100, whereby a user can manipulate the suction supply unit 300 with his or her thumb while holding the handle 100 and operating the switch 520 with his or her index finger, thereby improving manipulation convenience.

The electrode clip 530 may be electrically connected to the switch 520 in the installation space 115.

The electrode clip 530 may include a connection piece 531, an insertion piece 532, a contact piece 533, and a bending portion 535.

The connection piece 531 may be electrically connected to the switch 520 through an electric line (not shown).

The insertion piece 532 may be inserted into the slit 372 formed through the third connection port 370.

The contact piece 533 may extend from the insertion piece 532 and may be inserted into the accommodation flow channel 371 to contact the conductive tube 210 in the accommodation flow channel 371. The contact piece 533 may be formed at one end thereof with a contact facet 534 having a shape corresponding to an outer peripheral surface of the conductive tube 210. Accordingly, even upon rotation of the conductive tube 210, the contact facet 534 may be kept in a state of contacting the conductive tube 210, whereby electricity can be stably supplied to the conductive tube 210.

The bending portion 535 connects the connection piece 531 to the insertion piece 532. The bending portion 535 elastically supports the insertion piece 532 while pushing the insertion piece 532 towards the conductive tube 210 such that contact between the contact piece 533 and the conductive tube 210 can be maintained.

The second handle case 120 may include a first coupling socket 124, a second coupling socket 125, a first guide rib 126, a second guide rib 127, and a third guide rib 128. Although the following description will be given with reference to the second handle case 120, the structures of the sockets 124,125 and the guide ribs 126,127,128 may also be applied to the first handle case 110.

The first coupling socket 124 may be formed to receive the third coupling protrusion 374 of the third connection port 370 coupled thereto. In addition, the second coupling socket 125 may be formed to receive the first coupling protrusion 315 of the second chamber 320. With this structure, the suction supply unit 300 can be firmly secured to the first handle case 110 and the second handle case 120.

In addition, the first guide rib 126 may have a first space 126a and a second space 126b. The second guide rib 127 may have a third space 127a.

The supply tube 130 and the suction tube 140 may be coupled to the first connection port 350 and the second connection port 360 via the first space 126a and the third space 127a, respectively.

The electric line (not shown) may be disposed in a groove of the third guide rib 128 via the second space 126b and the third space 127a. As a result, in the installation space 115, the supply tube 130, the suction tube 140 and the electric line may be suitably arranged to provide a pleasant appearance.

This application was supported by the research project as below.
[Project number] 1415177299
[Ministry] MINISTRY OF TRADE, IDCUSTRY AND ENERGY
[Management agency] Korea Institute for Advancement of Technology
[Program name] Promotion of technical performance (R&D)
[Project name] Robotic or Laparoscopic Suction Irrigation System with Pressure Regulation
[Contribution ratio] 1/1
[Supervision institution] Orange Medics, Inc.
[Period] 2021.09.01 ~ 2022.08.31

Although some embodiments have been described herein, it should be understood that these embodiments are provided for illustration only and are not to be construed in any way as limiting the present invention. Further, it should be understood by those skilled in the art that various modifications, changes, and alterations can be made by those skilled in the art without departing from the spirit and scope of the present invention. For example, each component described as a single type may be implemented in a separate form, and components described as distributed may also be implemented in a combined form.
Therefore, the scope of the invention should be limited only by the accompanying claims and equivalents thereto.

**<List of Reference Numerals>**

| | | | |
|---|---|---|---|
| 100: | Handle | 110, 120: | Handle case |
| 200: | Cannula | 210: | Conductive tube |
| 220: | Electrode | 230: | Protective tube |
| 300: | Suction supply unit | 310: | First chamber |
| 320: | Second chamber | 330: | First rotary valve |
| 334: | First ring valve | 340: | Second rotary valve |
| 344: | Second ring valve | 370: | Third connection port |
| 372: | Slit | 400: | Manipulation unit |
| 410: | Adapter | 420: | First knob |
| 430: | Second knob | 440: | Insert |
| 500: | Switch unit | 530: | Electrode clip |

## Claims

1. A flow controllable type suction and irrigation device comprising:
a handle having an installation space therein;
a cannula provided with a conductive tube and an insulating protective tube, the conductive tube extending in a forward direction of the handle to be inserted into the abdominal cavity of a patient and being coupled to an electrode for surgery, the insulating protective tube being disposed to surround the conductive tube; and
a suction supply unit provided to the handle and supplying an irrigation fluid to the cannula or suctioning blood or contaminants from the abdominal cavity of the patient through the cannula,
wherein the suction supply unit comprises a first chamber having a first through-hole, a second chamber connected to the first chamber through a communication hole and having a second through-hole, a first rotary valve disposed inside the first chamber and regulating an open area of the first through-hole while rotating, and a second rotary valve disposed inside the second chamber and regulating an open area of the second through-hole while rotating.

2. The flow controllable type suction and irrigation device according to claim 1, wherein the first rotary valve comprises:
a first body spaced apart from an inner peripheral surface of the first chamber;
a first sealing ring surrounding a lower region of the first body;
a second sealing ring surrounding an upper region of the first body; and
a first ring valve disposed on the first sealing ring to protrude in a circumferential direction of the first body, the first ring valve being formed to closely contact the inner peripheral surface of the first chamber and regulating the open area of the first through-hole while rotating together with the first body.

3. The flow controllable type suction and irrigation device according to claim 2,
wherein the first rotary valve further comprises a first stationary flange protruding from a lower end of the first body in the circumferential direction of the first body, and a pair of second stationary flanges spaced apart from each other and protruding from the upper region of the first body in the circumferential direction of the first body;
wherein the first sealing ring is disposed between the first stationary flange and the first ring valve; and
wherein the second sealing ring is disposed between the pair of second stationary flanges.

4. The flow controllable type suction and irrigation device according to claim 2 or 3, wherein the first ring valve has a lower surface formed in the circumferential direction beneath a lower end of the first through-hole and an upper surface slanted upwards in a first rotation direction such that a height of the first ring valve gradually increases in the first rotation direction.

5. The flow controllable type suction and irrigation device according to claim 4, wherein the open area of the first through-hole opened by the first ring valve is increased as the first rotary valve is rotated in the first rotation direction, and is decreased as the first rotary valve is rotated in an opposite direction to the first rotation direction.

6. The flow controllable type suction and irrigation device according to any one of claims 1 to 5, wherein the suction supply unit comprises:
a first connection port connected to the first through-hole and extending in a backward direction of the first chamber to be connected to a supply tube;
a second connection port connected to the second through-hole and extending in a backward direction of the second chamber to be connected to a suction tube; and
a third connection port connected to the communication hole and extending in a forward direction of the first chamber and the second chamber to be connected to the cannula.

7. The flow controllable type suction and irrigation device according to claim 6, further comprising:
a switch unit provided to the handle and supplying electricity to the conductive tube, the switch unit comprising:
a stationary base coupled to the handle in the installation space and closely contacting the third connection port;
a switch provided to the stationary base, connected to an electric line through which electricity is supplied, and exposed outside the handle; and
an electrode clip electrically connected to the switch in the installation space and inserted into a slit formed through the third connection port to contact the conductive tube.

8. The flow controllable type suction and irrigation device according to claim 7, wherein the stationary base comprises a support flange formed corresponding to an outer peripheral surface of the third connection port and formed with a rounded section closely contacting the third connection port.

9. The flow controllable type suction and irrigation device according to claim 7 or 8, wherein the handle comprises a first handle case and a second handle case, and the switch unit is disposed biased to one side of the second handle case.

10. The flow controllable type suction and irrigation device according to claim 9, wherein the second handle case comprises:
an opening formed through the second handle case to expose the switch;
an upper shaft cover receiving an upper portion of a rotational shaft provided to the switch, wherein the rotational shaft constitutes a center of rotation of the switch; and
a guide wall formed along a circumference of the opening inside the second handle case.

11. The flow controllable type suction and irrigation device according to claim 10, wherein the stationary base comprises:
a coupling plate formed to closely contact the guide wall; and
a lower shaft cover formed on the coupling plate to receive a lower portion of the rotational shaft, and coupled to the upper shaft cover.

12. The flow controllable type suction and irrigation device according to any one of claims 6 to 11, further comprising:
an adapter rotatably provided to the third connection port and coupled to one end of the conductive tube therein;
a first knob disposed at a front side of the handle to reciprocate in an axial direction of the conductive tube and receiving a portion of the adapter in an interior space of the first knob;
a second knob coupled to a front end of the first knob and coupled to a rear end of the protective tube therein, the second knob being integrally operated with the first knob; and
a manipulation unit coupled to a rear end of the second knob and having an insert securing the rear end of the protective tube together with the second knob.

13. The flow controllable type suction and irrigation device according to claim 12, wherein the protective tube comprises a tube expansion portion formed at the rear end portion of the protective tube, the tube expansion portion being pressed and secured by the insert and a first pressing portion formed at a rear side of the second knob.

14. The flow controllable type suction and irrigation device according to any one of claims 1 to 13, wherein the suction supply unit further comprises:
a first turning head disposed at an upper end of the first rotary valve and transferring rotational force supplied from outside to the first rotary valve; and
a second turning head disposed at an upper end of the second rotary valve and transferring rotational force supplied from outside to the second rotary valve.
